# EUROPEAN PATENT APPLICATION

(11) **EP 1 967 181 A1**
(43) Date of publication of application: **10.09.2008**
(21) Application number: 07384018.3
(22) Date of filing: 06.03.2007
(51) Int. Cl.: A61K 9/14, A61K 9/16, A61K 31/4155

(54) **Pharmaceutical formulation comprising a CB1-Receptor compound in a solid solution and/or solid dispersion**

(71) Applicant: Laboratorios del Dr. Esteve S.A., 08041 Barcelona (ES)
(72) Inventor: Santanach Delisau, Angel., 08480 LÀmetlla del Vallés (ES); Casadevall Pujals, Gemma., 08019 Barcelona (ES)
(74) Representative: Peters, Hajo

(57) **Abstract**

The present invention relates to a pharmaceutical formulation comprising 5-(4-Chlorophenyl)-1-(2,4-dichloro-phenyl)-4,5-dihydro-1H-pyrazole-3-carboxylic acid piperidin-1-ylamide as racemate or (R)- and (S)-enantiomer or mixtures thereof in a solid solution and/or solid dispersion.

## Description

The present invention relates to a pharmaceutical formulation comprising 5-(4-Chlorophenyl)-1-(2,4-dichloro-phenyl)-4,5-dihydro-1H-pyrazole-3-carboxylic acid piperidin-1-ylamide as racemate or (R)- and (S)-enantiomer or mixtures thereof in a solid solution and/or solid dispersion.

Cannabinoids are compounds, which are derived from the cannabis sativa plant which is commonly known as marijuana. The most active chemical compound of the naturally occurring cannabinoids is tetrahydrocannabinol (THC), particularly Δ⁹-THC.

These naturally occuring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated CB₁ and CB₂ are involved in a variety of physiological or pathophysiological processes in humans and animals, e.g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992.

Therefore, compounds, which have a high binding affinity for these cannabinoid receptors and which are suitable for modulating these receptors are useful in the prevention and/or treatment of cannabinoid-receptor related disorders.

In particular, the CB₁-Receptor is involved in many different food-intake related disorders such as bulimia or obesity, including obesity associated with type II diabetes (non-insulin-dependent diabetes) and thus, compounds suitable for regulating this receptor may be used in the prophylaxis and/or treatment of these disorders.

Also dyslipidaemia, metabolic syndrome (both in its weight dependent or weight independent aspects) and diabetes type II have over the last decades risen in importance for the public health, especially for the developed or developing countries, likely due to a demoscopically marked increase in the proportion of obese or overweight members of the population or in average age.

The compounds according to formula (I), (Ia) or (Ib) (also known as 5-(4-Chloro-phenyl)-1-(2,4-dichloro-phenyl)-4,5-dihydro-1H-pyrazole-3-carboxylic acid piperidin-1-ylamide as racemate and its (S)- and (R)-enantiomers) are described together with ways for their synthesis, their melting point and their biological activity in applications WO 2005/077911 A1 (racemate), WO 2007/09686 A2 ((S)-enantiomer)and WO 2007/09695 A1 ((R)-enantiomer) respectively; the content of these publications being included here by reference. These compounds were shown to be CB1-binders and/or compounds with antiobesity activity and/or compounds having a reducing effect on triglyceride levels in blood in in-vivo models. Thus, these compounds according to formulas (I), (Ia) and (Ib) did show clear effects especially hinting at a great potential for the treatment or prevention of obesity, dyslipidaemia, metabolic syndrome (both in its weight dependent or weight independent aspects) and/or diabetes type II.

Thus, it was an object of the present invention to provide suitable pharmaceutical compositions for these compounds. As these compounds do have a tendency to be not highly soluble, this aspect - important when dealing with pharmaceutical compositions, especially those suitable to enhance the absorption of the active principle through a mucosa, especially for pharmaceutical compositions for oral administration - under certain therapeutical circumstances needs improvement.

Said object was achieved by providing pharmaceutical composition comprising a solid dispersion and/or solid solution according to the invention and optionally further pharmaceutical acceptable ingredients.

Thus in another related aspect the invention further provides a solid dispersion and/or solid solution of one or more active principles selected from compounds of formula (I), (Ia) or (Ib) or mixtures thereof 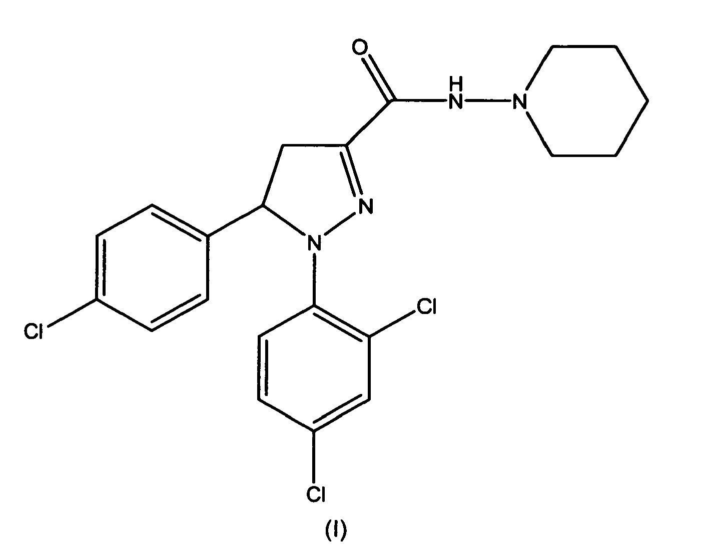 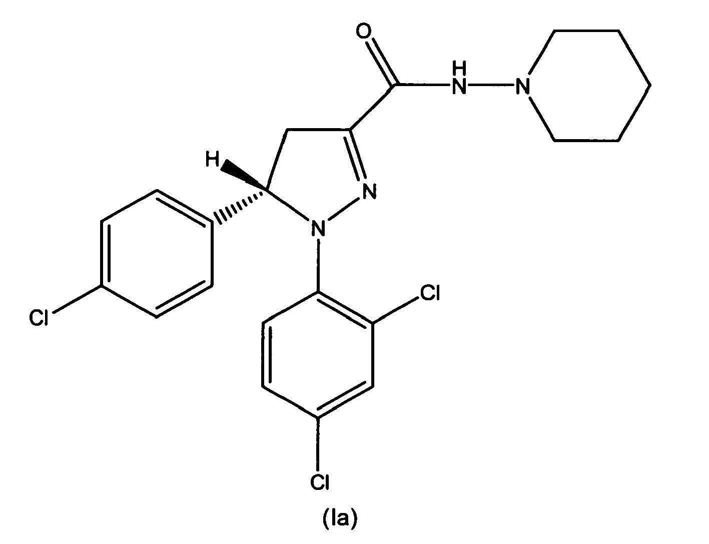 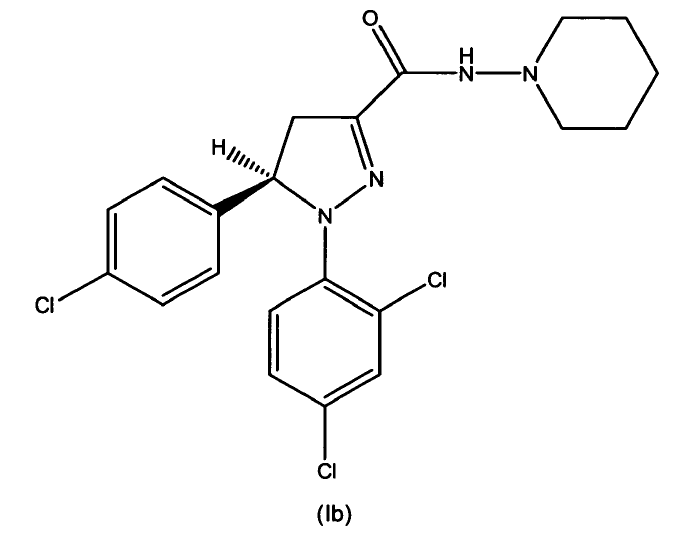 , optionally in form of one or more of the appropriate polymorphs, in amorphous form and/or corresponding salts and/or corresponding solvates thereof,
obtainable by a process in which
a) the active principle/s is/are mixed with an at least equimolar amount of at least one carrier,
b) thereafter or during step a) the mixture is heated until the mixture is melted and
c) subsequently the - preferably eutectic - mixture is cooled below the melting point.

It is found that the release and uptake of the active principle may be greatly enhanced and improved by the pharmaceutical compositions according to the invention. Especially these compositions do show an improved dissolution or dissolution profile, mostly expressed as a higher dissolution rate of the active principle (e.g. expressed as mg/hr). As this is positively influencing also the uptake of the active principle into the body, as a consequence the pharmaceutical composition is allowing for a lowered effective dose to be used, which thus clearly also acts positively on any unwanted side effect. On the other hand the composition according to the invention by its improved dissolution rate results also in a higher ratio of the active principle crossing the blood/brain barrier, creating faster the - in some cases - necessary concentrations of the active principle in the CNS, while at the same time allowing thus for an even further reduction of the amount of active principle having to be applied to the patient. In addition the formulations according to the invention are also thus especially useful for the treatment of acute abuse e.g. as an emergency treatment after abuse of a medicament/drug.

"Solid dispersion and/or solid solution" is defined according to the invention as a dispersion or solution of the active principle in an inert carrier or matrix at solid state prepared through melting with a carrier or coprecipitation/coevaporation from a solution with a carrier and a solvent. Preferably the active principle/s is/are finely dispersed/dissolved up to and including single molecules being dispersed/dissolved in the carrier. During the preparation by melting a physical mixture of at least one active principle is mixed with at least one carrier, melted and the melt rapidly solidified by lowering the temperature. During the preparation by coprecipitation/coevaporation from a solution carrier and active principle are dissolved in a solvent, mixed and the solvent removed (usually evaporated or maybe freezedried or spraydried). Most preferably according to the invention "solid dispersion and/or solid solution" is defined as a dispersion of the active principle in an inert carrier or matrix at solid state prepared through melting.

"Carrier" in the sense of this application is a substance in which one or more active principles may be dispersed/dissolved, preferably finely dispersed/dissolved up to and including single molecules being dispersed in the carrier. Preferably a carrier is defined by one or more, preferably most, more preferably all - where applicable - of the following criteria:
- be freely water-soluble with intrinsic rapid dissolution properties;
- be nontoxic and pharmacologically inert;
- be heat stable with low melting point (if melting is used for the preparation of the solid dispersion);
- be soluble in a variety of solvents and pass through a vitreous state upon solvent evaporation (if solvent evaporation is used for the preparation of the solid dispersion);
- be able to increase the aqueous solubility of at least on of the active principles;
- be chemically compatible with the drug and not form a strongly bonded complex with any of the active principles dispersed therein.
Further examples of carriers may be found in Ford, J.L.; Acta Helv. 1986, 61, 69-88, enclosed here by reference.

"Hot melt extrusion" is defined as an extrusion of the active principle/s together with the carrier/s in an extruder, where the carrier/s and active principle/s are mixed and melted before or during extrusion thus creating upon extrusion a solid dispersion and/or solid solution (see above). Hot-melt extrusion is described e.g. by Koleng at al. "Hot-Melt Extrusion Technology", Encyclopedia of Pharmaceutical Technology (2002), Macel Dekker, Inc.

The active principle according to the invention may be subject to any particle size reduction like milling, grinding, nanosizing or micronisations, or to complexation like e.g. complexation with Ciclodextrines, or PEG.

In a preferred embodiment of the solid dispersion and/or solid solution according to the invention the carrier is either hydrophilic or hygroscopic and has a melting point between +40°C and the melting point of the active principle or mixture of active principles +20 °C.

Preferably "hydrophilic" means in the context of this application that the carrier shows an HLB (hydrophilic-lipophilic balance) determined according to the method of W. C. Griffin (1954) of 12 to 20, more preferably 15 to 20.

In another preferred embodiment of the solid dispersion and/or solid solution according to the invention the carrier is selected from
- sugars, like dextrose, sucrose, galactose, sorbitol, maltose, xylitol, manitol, lactose;
- acids, like citric acid, succinic acid, ascorbic acid;
- polymeric materials, like povidone (PVP), polyethylene oxide (PEO), polyethylene glycol (PEG), hydroxpropylmethylcellulose (HPMC), methylcellulose (MC), ethylcellulose (EC), hydroxyethylcelllose (HEC), cylodextrin, hydroxypropylcellulose (HPC), pectin, galactomannan;
- insoluble or enteric polymers, like hydroxypropylmethylcellulose phthalate, polymethacrylates (e.g. Eudragit L-100, Eudragit S-100, Eudragit RL, Eudragit RS);
- surfactants, like polyoxyethylene stearate, renex, poloxamer 188, texafor, AIP, deoxycholic acid, polyoxy-ethylene-sorbitan higher fatty acid esters (e.g. Tween, like Tween 80), spans;
- others, consisting of: camuba wax, pentaerythritol, pentaerythrityltetraacetate, urea, urethane, hydroxyalkylxanthins;
preferably the carriers are selected from EUDRAGIT, MYRJ 52, VITE-TPGS, GELUCIRE 50/13, HPMC-PHTALATE, HPMC, HEC, HPC-SL, PEO and/or POLOXAMER.

In another preferred embodiment of the solid dispersion and/or solid solution according to the invention
- the melting temperature in step (b) is in the range between +40°C and the melting point of the active principle or mixture of active principles +20 °C; and/or
- the cooling step (c) is done by lowering the temperature by more than 10°C/sec, preferably 20°C/sec, down to temperatures below 25°C, preferably below 0 °C; and/or is done by contacting the melt of step (b) with an environment having a temperature of 25°C or lower, preferably 0°C or lower.

In another preferred embodiment of the solid dispersion and/or solid solution according to the invention the molecular ratio between carrier and active principle is between 1:1 and 1: 20, preferably is between 1:1 and 1:10, more preferably is between 1:2 and 1:5.

In a preferred embodiment of the pharmaceutical composition according to the invention
- the active principle is present in an amount of 10 to 60, preferably 20 to 40 % by weight based on the total weight of the composition;
   and/or
- the active principle is present in an amount of 1 to 250 mg, preferably 10 to 200 mg, more preferably 15 to 150 mg in the composition.

In another preferred embodiment of the pharmaceutical composition according to the invention it is suitable to enhance the absorption of the active principle through a mucosa.
Thereby it is preferred if the mucosa is selected from the
- nasal or olfactory mucosa,
- buccal or oral mucosa,
- gastric mucosa,
- intestinal mucosa
- vaginal mucosa, or
- rectal mucosa,
preferably selected from
- gastric mucosa, or
- intestinal mucosa.

In another preferred embodiment of the pharmaceutical composition according to the invention the pharmaceutical composition is selected from
- a pharmaceutical composition for oral application,
- a pharmaceutical composition for nasal application,
- a pharmaceutical composition for buccal application,
- a pharmaceutical composition for rectal application, or
- a pharmaceutical composition for vaginal application;
or
- a pharmaceutical composition for transdermal application;
- a pharmaceutical composition for systemic application;
preferably selected from
- a pharmaceutical composition for oral application.

In another preferred embodiment of the pharmaceutical composition according to the invention the pharmaceutical composition is selected from
- a tablet,
- a capsule,
- a sachet,
- a powder,
- a caplet,
- a gel,
- a film,
- a pellet,
- a granule,
- an implant,
- a multiparticulate with granules compressed into a tablet,
- a multiparticulate with granules filled into a capsule,
- a multiparticulate with pelets compressed into a tablet,
- a multiparticulate with pelets filled into a capsule, or
- a suppository,
or
- an injectable solution/dispersion
- a transdermal system like a patch;
preferably selected from
- a tablet,
- a capsule,
- a pellet,
- a granule,
- a multiparticulate with granules compressed into a tablet,
- a multiparticulate with granules filled into a capsule,
- a multiparticulate with pelets compressed into a tablet, or
- a multiparticulate with pelets filled into a capsule.

In another preferred embodiment of the pharmaceutical composition according to the invention
- the further pharmaceutical acceptable ingredients are present in a total amount of 5 to 95 %, preferably 50 to 90 % by weight based on the total weight of the composition;
   and/or
- the further pharmaceutical acceptable ingredients are present in a total amount of 0 to 300 mg, preferably 0 to 250 mg, more preferably 20 to 250 mg.

In another preferred embodiment of the pharmaceutical composition according to the invention the further pharmaceutical acceptable ingredient/s is/are selected from a diluent, a binder and/or a lubricant, and/or optionally a flowing agent, an antiadhesive, a preservative, and/or, optionally, a taste masking agent, a coloring agent and/or a flavoring agent and/or a permeation enhancer.

In another preferred embodiment of the pharmaceutical composition according to the invention the further composition is prepared using hot-melt extrusion.

Another preferred aspect of the invention is a pharmaceutical composition comprising as active principle at least one of
- (rac)-5-(4-Chloro-phenyl)-1-(2,4-dichloro-phenyl)-4.5-dihydro-1H-pyrazole-3-carboxylic acid piperidin-1-ylamide;
- (R)- 5-(4-Chloro-phenyl)-1-(2,4-dichloro-phenyl)-4,5-dihydro-1H-pyrazole-3-carboxylic acid piperidin-1-ylamide, or
- (S)- 5-(4-Chloro-phenyl)-1-(2,4-dichloro-phenyl)-4,5-dihydro-1H-pyrazole-3-carboxylic acid piperidin-1-ylamide,
- or mixtures thereof;
   optionally in form of one or more of the appropriate polymorphs, in amorphous form and/or corresponding salts and/or corresponding solvates thereof,
   in a solid dispersion and/or solid solution in a carrier, the carrier having a melting point between +40°C and the melting point of the active principle +20 °C and being hydrophilic or hygroscopic;
and optionally further pharmaceutical acceptable ingredients

Preferred concentration/weight/percentage ranges, forms of the compositions, as well as appropriate lists of preferred excipients or carriers have already been described above.

In a preferred embodiment of any of the pharmaceutical compositions or the solid dispersion according to the invention described above the active principle is selected from
a) (rac)-5-(4-Chloro-phenyl)-1-(2,4-dichloro-phenyl)-4,5-dihydro-1H-pyrazole-3-carboxylic acid piperidin-1-ylamide;
   preferably in
   - form of the α-polymorph;
   - form of the β-polymorph;
   - amorphous form;
   - form of a solvate, preferably a hydrate, more preferably a monohydrate;
   - the free base; or
   - a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1 ,5-disulfonic acid, nitric acid, phosophoric acid, *p*-toluenesulfonic acid, sulfuric acid and/or thiocyanic acid; more preferably the salt being a hydrochloride;
b) (R)- 5-(4-Chloro-phenyl)-1-(2,4-dichloro-phenyl)-4,5-dihydro-1H-pyrazole-3-carboxylic acid piperidin-1-ylamide, preferably
   preferably in
   - form of the α-polymorph;
   - form of the γ-polymorph;
   - amorphous form;
   - form of a solvate, preferably a hydrate,
   - the free base;
   - a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, p-toluenesulfonic acid, sulfuric acid and/or thiocyanic acid; more preferably the salt being a hydrochloride;
c) (S)- 5-(4-Chloro-phenyl)-1-(2,4-dichloro-phenyl)-4,5-dihydro-1H-pyrazole-3-carboxylic acid piperidin-1-ylamide,
   preferably in
   - form of a polymorph;
   - amorphous form;
   - form of a solvate, preferably a hydrate,
   - the free base;
   - a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1 ,5-disulfonic acid, nitric acid, phosophoric acid, *p*-toluenesulfonic acid, sulfuric acid and/or thiocyanic acid; more preferably the salt being a hydrochloride;
   or
d) a nonracemic mixtures of (b) and (c).

The γ-polymorph form of (rac)-5-(4-Chloro-phenyl)-1-(2,4-dichloro-phenyl)-4,5-dihydro-1H-pyrazole-3-carboxylic acid piperidin-1-ylamide including methods for its production and its melting point is described and exemplified in WO07/17126, the content of which is herwewith enclosed in its entirety by reference.The amorphous form of (rac)-5-(4-Chloro-phenyl)-1-(2,4-dichloro-phenyl)-4,5-dihydro-1H-pyrazole-3-carboxylic acid piperidin-1-ylamide including methods for its production and its melting point is described and exemplified in WO07/17124, the content of which is herwewith enclosed in its entirety by reference. A hydrate, especially the mono-hydrate of (rac)-5-(4-Chloro-phenyl)-1-(2,4-dichloro-phenyl)-4,5-dihydro-1H-pyrazole-3-carboxylic acid piperidin-1-ylamide including methods for its production and its melting point is described and exemplified in WO07/09705, the content of which is herwewith enclosed in its entirety by reference.

Salts with an acid with a pkₐ ≤ 3.0 of (rac)-5-(4-Chloro-phenyl)-1-(2,4-dichloro-phenyl)-4,5-dihydro-1H-pyrazole-3-carboxylic acid piperidin-1-ylamide (R)-5-(4-Chloro-phenyl)-1-(2,4-dichloro-phenyl)-4,5-dihydro-1H-pyrazole-3-carboxylic acid piperidin-1-ylamide and (S)-5-(4-Chloro-phenyl)-1-(2,4-dichioro-phenyl)-4,5-dihydro-1H-pyrazole-3-carboxylic acid piperidin-1-ylamide and their melting points are described and exemplified in WO07/09708 the content of which is herwewith enclosed in its entirety by reference. Specifically mentioned are salts of the the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, p-toluenesulfonic acid, sulfuric acid and/or thiocyanic acid. Some salts like e.g. hydrochloride salts etc. of (rac)-5-(4-Chloro-phenyl)-1-(2,4-dichlorophenyl)-4,5-dihydro-1H-pyrazole-3-carboxylic acid piperidin-1-ylamide, (R)-5-(4-Chloro-phenyl)-1-(2,4-dichloro-phenyl)-4,5-dihydro-1H-pyrazole-3-carboxylic acid piperidin-1-ylamide and (S)-5-(4-Chloro-phenyl)-1-(2,4-dichloro-phenyl)-4,5-dihydro-1H-pyrazole-3-carboxylic acid piperidin-1-ylamide have also been described in their respective applications WO 2005/077911 A1, WO 2007/09695 A1 and WO 2007/09686 A2.

The α-polymorph of (rac)-5-(4-Chloro-phenyl)-1-(2,4-dichloro-phenyl)-4,5-dihydro-1H-pyrazole-3-carboxylic acid piperidin-1-ylamide is defined by its physico-chemical attributes from the following list:
- it crystallizes as a triclinic cell with an α-angle of 78° and/or a β-angle of 74° and a γ-angle of 88°;
- it melts
   above 180°C, preferably at an onset point of 183 ± 1 °C, more preferably at an onset point of 183-184 °C
   and/or
   with a melting peak at 185 ± 2 °C, preferably at 184 ± 1 °C;
- it decomposes at or above 280 °C;
- it shows in the FTRaman spectrum bands, preferably intense bands, at 2932 ± 5 cm⁻¹ and/or 1647 ± 5 cm⁻¹ and/or 1593 ± 5 cm⁻¹ and/or 1394 ± 5 cm⁻¹ and/or 1269 ± 5 cm⁻¹ and/or 1253 ± 5 cm⁻¹ and/or 1109 ± 5 cm⁻¹ and/or 1084 ± 5 cm⁻¹ and/or 1047 ± 5 cm⁻¹ and/or 783 ± 5 cm⁻¹ and/or 661 ± 5 cm⁻¹ and/or a Raman shift at 1253 ± 5 cm⁻¹ and/or at 1084 ± 5 cm⁻¹ and/or at 533 ± 5 cm⁻¹ and/or and at 364 cm⁻¹

In the process for the manufacture of the α-Polymorph of (rac)-N-piperidinyl-5-(4-chlorophenyl)-1-(2,4-dichlorophenyl)-4,5-dihydro-1H-pyrazole-3-carboxamide a solution of (rac)-N-piperidinyl-5-(4-chlorophenyl)-1-(2,4-dichlorophenyl)-4,5-dihydro-1H-pyrazole-3-carboxamide in an alcohol; preferably ethanol or methanol; is evaporated at room temperature (25 °C) to yield the α-Polymorph of (rac)-N-piperidinyl-5-(4-chlorophenyl)-1-(2,4-dichlorophenyl)-4,5-dihydro-1H-pyrazole-3-carboxamide.

The α-Polymorph of (R)-N-piperidinyl-5-(4-chlorophenyl)-1-(2,4-dichlorophenyl)-4,5-dihydro-1H-pyrazole-3-carboxamide is defined by its physico-chemical attributes from the following list:
- it crystallizes as a monoclinic cell with a beta angle of 104.52° at 100 K;
- it melts
   above 114°C, preferably at an onset point of 118 ± 4 °C, more preferably at an onset point of 118 ± 3 °C
   and/or
   with a melting peak at 124 ± 6°C, preferably at 124 ± 5°C,
   and/or
   with a fusion enthalpy of -58 ± 4. J/g, preferably -58 ± 3.0 J/g;
- it decomposes at or above 280 °C;
- it shows in the FTIR spectrum peaks, preferably intense peaks, at 3324 ± 5 cm⁻¹ and/or 1534 ± 5 cm⁻¹;
- it shows in the FTRaman spectrum bands, preferably intense bands, at 3326 ± 5 cm⁻¹ and/or a Raman shift at 3326 ± 5 cm⁻¹.

The process for the manufacture of the α-Polymorph of (R)-N-piperidinyl-5-(4-chlorophenyl)-1-(2,4-dichlorophenyl)-4,5-dihydro-1H-pyrazole-3-carboxamide involves the following steps:
a) in a solution step (R)-N-piperidinyl-5-(4-chlorophenyl)-1-(2,4dichlorophenyl)-4,5-dihydro-1H-pyrazole-3-carboxamide is dissolved in diisopropyl ether, heated to 68°C until (R)-N-piperidinyl-5-(4-chlorophenyl)-1-(2,4-dichlorophenyl)-4,5-dihydro-1H-pyrazole-3-carboxamide is complete dissolved, and
b) in a cooling step done in two phases the solution first is subsequently cooled to 20 ± 2 °C, and then to 0 ± 2 °C, to yield the α-Polymorph of (R)-N-piperidinyl-5-(4-chlorophenyl)-1-(2,4-dichlorophenyl)-4,5-dihydro-1H-pyrazole-3-carboxamide.

The amorphous phase of (R)-N-piperidinyl-5-(4-chlorophenyl)-1-(2,4-dichlorophenyl)-4,5-dihydro-1H-pyrazole-3-carboxamide is defined by its physico-chemical attributes from the following list:
- it shows a glass transition in the DSC analysis at 64.5 ± 4 °C, preferably at 64.5 ± 3 °C, more preferably at 64.5 ± 2 °C or also between 63 and 66° C;
- it decomposes at or above 300 °C;
- it shows in the FTIR spectrum peaks, preferably intense and/or broad peaks at 1656 ± 5 cm⁻¹ and/or 1473 ± 5 cm⁻¹ and no peaks, preferably no intense peaks, at 3325 ± 10 cm⁻¹;
- it shows in the FTRaman spectrum bands, preferably intense and/or broad bands, at 1666 ± 5 cm⁻¹.

In the process for the manufacture of the amorphous phase of (R)-N-piperidinyl-5-(4-chlorophenyl)-1-(2,4-dichlorophenyl)-4,5-dihydro-1H-pyrazole-3-carboxamide (R)-N-piperidinyl-5-(4-chlorophenyl)-1-(2,4dichlorophenyl)-4,5-dihydro-1H-pyrazole-3-carboxamide is melted, preferably at a temperature between 78 and 88°C, and subsequently cooled to below the melting point, preferably to room temperature.

The γ-Polymorph of (R)-N-piperidinyl-5-(4-chlorophenyl)-1-(2,4-dichlorophenyl)-4,5-dihydro-1H-pyrazole-3-carboxamide is defined by its physico-chemical attributes from the following list:
- it crystallizes as a monoclinic cell with a beta angle of 92.15° at room temperature and/or a beta angle of 92.41° at 100 K;
- it melts
   above 120°C, preferably at an onset point of 122 ± 2 °C, more preferably at an onset point of 122 ± 1 °C,
   and/or
   with a melting peak at 127 ± 3 °C, preferably at 127 ± 2 °C, more preferably at 127 ± 1.5°C.
   and/or
   with a fusion enthalpy of -62.5 ± 3.0 J/g, preferably -62.5 ± 2.0 J/g, or preferably -62.5 ± 1.3 J/g;
- it decomposes at or above 299 °C;
- it shows in the FTIR spectrum peaks, preferably intense peaks, at 1665 ± 5 cm⁻¹ and no peaks, preferably no intense peaks, at 3325 ± 10 cm⁻¹;
- it shows in the FTRaman spectrum bands, preferably intense bands, at 1666 ± 5 cm⁻¹ and/or 1585 ± 5 cm⁻¹ and/or a Raman shift at 1666 ± 5 cm⁻¹.

The process for the manufacture of the y-Polymorph of (R)-N-piperidinyl-5-(4-chlorophenyl)-1-(2,4-dichlorophenyl)-4,5-dihydro-1H-pyrazole-3-carboxamide involves the following steps:
a) in a solution step (R)-N-piperidinyl-5-(4-chlorophenyl)-1-(2,4dichlorophenyl)-4,5-dihydro-1H-pyrazole-3-carboxamide is dissolved in diisopropyl ether, heated to 68°C until (R)-N-piperidinyl-5-(4-chlorophenyl)-1-(2,4-dichlorophenyl)-4,5-dihydro-1H-pyrazole-3-carboxamide is complete dissolved, and
b) in a cooling step done in two phases the solution first is subsequently cooled to 20 ± 2 °C, and then to 0 ± 2 °C, to yield the γ-Polymorph of (R)-N-piperidinyl-5-(4-chlorophenyl)-1-(2,4-dichlorophenyl)-4,5-dihydro-1H-pyrazole-3-carboxamide.

The daily dosage for humans and animals may vary depending on factors that have their basis in the respective species or other factors, such as age, sex, weight or degree of illness and so forth. The daily dosage for humans may preferably be in the range from 10 to 400, preferably 15 to 300, more preferably 25 to 250 milligrams of active substance to be administered during one or several intakes per day. Most preferably is a dosage of 25 to 250 mg of active principle in one dosage unit.

The pharmaceutical acceptable ingredients excipients which optionally may be included in the composition according to the present invention include especially a diluent, a binder and/or a lubricant, wheras a flowing agent, a preservative, an antiadhesive and, optionally, a taste masking agent and a coloring agent and/or a flavoring agent as well as one or more permeation enhancers can also be added.

The diluent - if used - in the composition of the present invention can be one or more compounds which are capable of densifying the active principle to give the desired mass. The preferred diluents are inorganic phosphates such as calcium phosphates; sugars such as hydrated or anhydrous lactose, or mannitol; and cellulose or cellulose derivatives such as, for example, microcrystalline cellulose, starch, corn starch or pregelatinized starch. Lactose monohydrate, mannitol, microcrystalline cellulose and corn starch, used by themselves or in a mixture, for example a mixture of lactose monohydrate and corn starch, are very particularly preferred. The diluent - if present at all - is present in a proportion of 5 % to 90 % by weight based on the total weight of the composition according to the invention

The binder - if employed - in the composition of the present invention can be one or more compounds which are capable of densifying the active principle within the overall e.g. granular formulation by converting it to larger and denser particles with improved solid characteristics. The preferred binders are alginic acid or sodium alginate; cellulose and cellulose derivatives such as sodium carboxymethyl cellulose, ethyl cellulose, hydroxyethyl cellulose, hydroxypropyl cellulose, hydroxypropyl methyl cellulose or methyl cellulose; gelatin; acrylic acid polymers; and povidone, for example povidone K 30, which is a very particularly preferred binder. The binder - if present at all - is present in a proportion of 1% to 30% by weight based on the total weight of the composition according to the invention.

The lubricant - if employed - in the composition of the present invention can be one or more compounds which are capable of preventing the problems associated with the preparation of the dosage form, such as the sticking and/or seizing problems which occur in the machines during compression or filling. The preferred lubricants are fatty acids or fatty acid derivatives such as calcium stearate, glyceryl monostearate, glyceryl palmitostearate, magnesium stearate, sodium laurylsulfate, sodium stearylfumarate, zinc stearate or stearic acid; hydrogenated vegetable oils, for example hydrogenated castor oil; polyalkylene glycols, especially polyethylene glycol; sodium benzoate; or talcum. Magnesium stearate is preferred according to the present invention. The lubricant - if present at all - is present in a proportion of 0.2% to 5% by weight based on the total weight of the composition according to the invention.

The antiadhesive which may be employed in the composition of the present invention can be one or more compounds which are capable of reducing the sticky character of the formulation, for example of preventing adhesion to metal surfaces. The preferred antiadhesives are compounds containing silicon, for example silica or talcum. The antiadhesive - if present at all - can be present in a proportion of 0 to 5% by weight in the pharmaceutical composition according to the invention.

The flowing agent which may be employed in the composition of the present invention can be one or more compounds which are capable of facilitating the flow of the prepared formulation. The preferred flowing agents are compounds containing silicon, for example anhydrous colloidal silica or precipitated silica. The flowing agent - if present at all - can be present in a proportion of 0 to 15% by weight in the pharmaceutical composition according to the invention.

The permeation enhancer which may be employed in the composition of the present invention, especially if the composition is a transdermal formulation, can be one or more compounds which are capable of facilitating the permeation of the active principle from the composition trough a biological barrier like e.g. the skin. Preferred permeation enhancers are high-boiling alcohols, diols, fatty acid esters, oleic acid and glyceride-based solvents, like for example isobutyl or isopropyl alcohol. Other permeation enhancers include linoleic acid, oleic acid, dimethylacetamide, lauric acid, myristic acid, palmitic acid, dimethyl sulfoxide, glycofurol, thymol, pyrrolidone, macrogol 15 hydroxystearate; mineral oil, light; polyoxyethylene alkyl ethers, menthol, polycarbophil, isopropyl myristate, glyceryl monooleate, ethyl acetate, polyethylene glycol, carbomer. The permeation enhancer/s - if present at all - can be present in a proportion of 0 to 25% by weight in the pharmaceutical composition according to the invention.

According to the present invention, the pharmaceutical compositions may preferably be prepared by a direct compression process, a granulation, extrusion and further spheronization, or by a layering process into for example nonpareil seeds.

Thus, for the direct compression process the active principle in a solid solution and/or solid dispersion and the optional diluent, binder are combined by geometrical mixing and further mixed with other optional excipients such colorants or taste masking agents, afterwards the mixture can be either compressed, encapsulated or dosified into sachets or any other unidose system.

For the dry granulation process the internal phase, thus the active principle in a solid solution and/or solid dispersion, the optional diluent, the optional binder, and, optionally, the coloring agent are mixed optionally at room temperature. The ingredient or ingredients of the optional external phase, namely the optional lubricant, possibly the antiadhesive, the flowing agent and, if appropriate, the coloring agent and/or the flavoring agent, are then added to the graded dry grains.

For the extrusion-espheronization process, a mass comprising the active principle in a solid solution and/or solid dispersion - for example the one obtained in the previous pharagraph - is passed through a pharmaceutical extruder and further spheronized until obtaining multiparticulates containing the active principle of a desired particle size. Later on these particles are dried and susceptible to either encapsulation or added into sachets (adding lubricants and other required processing agents), compressed (if mixed with the proper compression base) or coated for further manipulation, e.g. further layering (see below).

In one embodiment of the layering process to an inert sugar/starch/cellulose spherical core, a layer is applied containing a mixture of the active principle in a solid solution and/or solid dispersion, the optional diluent, the optional binder, and, optionally, the coloring agent, optionally followed by a another - e.g. isolation - layer formed by water soluble polymers and compatible excipients. Finally, optionally a layer consisting of a controlled release coating - like e.g. an enteric coating - is applied.

In another possibility of the layering process to a spherical core comprising already the active principle in a solid solution and/or solid dispersion produced by e.g. the extrusion-spheronization process described above, optionally one or more futher layers are applied containing a mixture of e.g. the optional diluent, the optional binder, and, optionally, the coloring agent, optionally water soluble polymers and compatible excipients. Finally, optionally a layer consisting of a controlled release coating - like e.g. an enteric coating - is applied.

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

In another preferable aspect of the invention the pharmaceutical composition according to the invention is suitable for the modulation (regulation) of cannabinoid-receptors, preferably cannabinoid 1 (CB₁) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Particularly preferably said pharmaceutical composition is suitable for the prophylaxis and/or treatment of psychosis and also depression as well as memory disorders.

Also particularly preferably said pharmaceutical composition is suitable for the prophylaxis and/or treatment of neuropathic pain, hyperalgesia or allodynia.

Also particularly preferably said pharmaceutical composition is suitable for the prophylaxis and/or treatment of food intake disorders, preferably bulimia, anorexia, cachexia, obesity and/or type II diabetus mellitus (non-insuline dependent diabetes mellitus), more preferably obesity and/or type II diabetus mellitus (non-insuline dependent diabetes mellitus). The inventive medicament also seems to be active in the prophylaxis and/or treatment of appetency disorders, e.g. the pyrazoline compounds of general formula I also reduce the desire for sweets and other macronutrients. In addition said pharmaceutical composition is also suitable for the prophylaxis and/or treatment of metabolic syndrome (both in its weight dependent or weight independent aspects) and dyslipidaemia.

Also particularly preferably said pharmaceutical composition is suitable for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

Particularly preferably said pharmaceutical composition is suitable for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Medicaments and/or drugs, which are frequently the subject of misuse include opioids, barbiturates, cannabis, cocaine, amphetamines, phencyclidine, hallucinogens and benzodiazepines. The formulations according to the invention are especially useful for the treatment of acute abuse e.g. as an emergency treatment after abuse of any of the medicaments/drugs mentioned above.

The pharmaceutical composition according to the invention is also suitable for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

In an embodiment of the pharmaceutical composition according to the invention the medicament is for the regulation of triglyceride levels in the blood plasma and for the prophylaxis and/or treatment of disorders of the central nervous system, especially stroke, of disorders of the cardiovascular system and of of food intake disorders, preferably bulimia, anorexia, cachexia, obesity, type II diabetus mellitus (non-insuline dependent diabetes mellitus), preferably obesity and diabetis.

In an embodiment of the pharmaceutical composition according to the invention the composition is for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

In an embodiment of the pharmaceutical composition according to the invention the composition is for the modulation of cannabinoid-receptors, preferably cannabinoid 1 (CB₁) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

The following part of the description is exemplifying the invention and is not meant to limit it.

### Examples:

### Example 1: (solid solution)

90 mg of the active principle (rac)-5-(4-Chloro-phenyl)-1-(2,4-dichloro-phenyl)-4,5-dihydro-1H-pyrazole-3-carboxylic acid piperidin-1-ylamide is mixed with 210 mg Eudragit L30D. The mixture is melted and after melting instantly cooled in an ice bath. The solid solution/dispersion can then be formulated with excipient by granulation into pellets or compressed into tablets.

| **FINAL COMPOSITION** | **mg** | **%** |
|---|---|---|
| (rac)-5-(4-Chloro-phenyl)-1-(2,4-dichloro-phenyl)-4,5-dihydro-1H- | | |
| pyrazole-3-carboxylic acid piperidin-1-ylamide (Pellets) | 90 | 30 |
| Eudragit L30D | 210 | 70 |

### Example 2: (hot melt extrusion)

90 mg of the active principle (rac)-5-(4-Chloro-phenyl)-1-(2,4-dichloro-phenyl)-4,5-dihydro-1H-pyrazole-3-carboxylic acid piperidin-1-ylamide is mixed with 180 mg Eudragit L30D and 30 mg of Vitamin E and subsequently melted and extruded in a holt-melt extruder. The solid solution/dispersion can then be formulated with excipient by granulation into pellets or compressed into tablets.

| **FINAL COMPOSITION** | **mg** | **%** |
|---|---|---|
| (rac)-5-(4-Chloro-phenyl)-1-(2,4-dichloro-phenyl)-4,5-dihydro-1H- | | |
| pyrazole-3-carboxylic acid piperidin-1-ylamide (Pellets) | 90 | 30 |
| Eudragit L30D | 180 | 60 |
| Vitamin E | 30 | 10 |

### Example 3 (Comparative example-Tablet):

1) Granulation step: In a high shear granulator the following excipients are mixed :
   - The active principle (rac)-5-(4-Chloro-phenyl)-1-(2,4-dichloro-phenyl)-4,5-dihydro-1H-pyrazole-3-carboxylic acid piperidin-1-ylamide,
   - Lactose
   - Maize starch-part
   The blend is mixed for 2 minutes and then, while under chopper and mixer action a solution containing PVP K 30 is incorporated. The mixture is granulated and the granulator unloaded.
2) Sieving (1): The final product is sieved to avoid agglomerates.
3) Drying: The final product from step 3 is dried using a fluid bed at 50°C until relative humidity is below 2%.
4) Blending: External phase excipients are added to the dry granule (Maize starch-part; SLS, CMC and Mg Stearate)
5) Sieving (2): The final product is sieved to avoid agglomerates.
6) Compression step:

| **FINAL COMPOSITION** | **%** |
|---|---|
| (rac)-5-(4-Chloro-phenyl)-1-(2,4-dichloro-phenyl)-4,5-dihydro-1H- | |
| pyrazole-3-carboxylic acid piperidin-1-ylamide | 28,4 |
| Maize Starch | 22,7 |
| Lactose Monohydrate | 42,6 |
| PVP K30 | 2,8 |
| SLS | 0,1 |
| CMC | 2,6 |
| Mg Stearate | 0,9 |

### Compressed as tablets.

### Example 4 (Comparative example-Non-excipients):

90 mg of the active principle (rac)-5-(4-Chloro-phenyl)-1-(2,4-dichloro-phenyl)-4,5-dihydro-1H-pyrazole-3-carboxylic acid piperidin-1-ylamide was placed into #1 hard gelatin capsules.

### Example 5: (solid solution, (R)-enantiomer

90 mg of the active principle (R)-5-(4-Chloro-phenyl)-1-(2,4-dichloro-phenyl)-4,5-dihydro-1H-pyrazole-3-carboxylic acid piperidin-1-ylamide is mixed with 210 mg Eudragit L30D. The mixture is melted and after melting instantly cooled in an ice bath. The solid solution/dispersion can then be formulated with excipient by granulation into pellets or compressed into tablets.

| **FINAL COMPOSITION** | **mg** | **%** |
|---|---|---|
| (R)-5-(4-Chloro-phenyl)-1-(2,4-dichloro-phenyl)-4,5-dihydro-1H- | | |
| pyrazole-3-carboxylic acid piperidin-1-ylamide (Pellets) | 90 | 30 |
| Eudragit L30D | 210 | 70 |

### Example 6: (hot melt extrusion, (R)-enantiomer

90 mg of the active principle (R)-5-(4-Chloro-phenyl)-1-(2,4-dichloro-phenyl)-4,5-dihydro-1H-pyrazole-3-carboxylic acid piperidin-1-ylamide is mixed with 180 mg Eudragit L30D and 30 mg of Vitamin E and subsequently melted and extruded in a holt-melt extruder. The solid solution/dispersion can then be formulated with excipient by granulation into pellets or compressed into tablets.

| **FINAL COMPOSITION** | **mg** | **%** |
|---|---|---|
| (R)-5-(4-Chloro-phenyl)-1-(2,4-dichloro-phenyl)-4,5-dihydro-1H- | | |
| pyrazole-3-carboxylic acid piperidin-1-ylamide (Pellets) | 90 | 30 |
| Eudragit L30D | 180 | 60 |
| Vitamin E | 30 | 10 |

### Example 7: (solid solution, (S)-enantiomer)

90 mg of the active principle (S)-5-(4-Chloro-phenyl)-1-(2,4-dichloro-phenyl)-4,5-dihydro-1H-pyrazole-3-carboxylic acid piperidin-1-ylamide is mixed with 210 mg Eudragit L30D. The mixture is melted and after melting instantly cooled in an ice bath. The solid solution/dispersion can then be formulated with excipient by granulation into pellets or compressed into tablets.

| **FINAL COMPOSITION** | **mg** | **%** |
|---|---|---|
| (S)-5-(4-Chloro-phenyl)-1-(2,4-dichloro-phenyl)-4,5-dihydro-1H- | | |
| pyrazole-3-carboxylic acid piperidin-1-ylamide (Pellets) | 90 | 30 |
| Eudragit L30D | 210 | 70 |

### Example 8: (hot melt extrusion, (S)-enantiomer)

90 mg of the active principle (S)-5-(4-Chloro-phenyl)-1-(2,4-dichloro-phenyl)-4,5-dihydro-1H-pyrazole-3-carboxylic acid piperidin-1-ylamide is mixed with 180 mg Eudragit L30D and 30 mg of Vitamin E and subsequently melted and extruded in a holt-melt extruder. The solid solution/dispersion can then be formulated with excipient by granulation into pellets or compressed into tablets.

| **FINAL COMPOSITION** | **mg** | **%** |
|---|---|---|
| (S)-5-(4-Chloro-phenyl)-1-(2,4-dichloro-phenyl)-4,5-dihydro-1H- | | |
| pyrazole-3-carboxylic acid piperidin-1-ylamide (Pellets) | 90 | 30 |
| Eudragit L30D | 180 | 60 |
| Vitamin E | 30 | 10 |

### IN-VITRO TESTS:

### In-vitro dissolution:

The samples are measured for the dissolution rate.

### STABILITY TESTS:

The samples according to the examples are tested for stability.

### COMPOUND EXAMPLES:

### Compound Example C1:

### α-Polymorph of (rac)-N-piperidinyl-5-(4-chlorophenyl)-1-(2,4-dichlorophenyl)-4,5-dihydro-1H-pyrazole-3-carboxamide

### C1(a):

The α-Polymorph was obtained by evaporation of a solution of N-piperidinyl-5-(4-chlorophenyl)-1-(2,4-dichlorophenyl)-4,5-dihydro-1H-pyrazole-3-carboxamide in ethanol at room temperature (25°C).

### C1(b):

The α-Polymorph was obtained by evaporation of a solution of N-piperidinyl-5-(4-chlorophenyl)-1-(2,4-dichlorophenyl)-4,5-dihydro-1H-pyrazole-3-carboxamide in methanol at room temperature (25°C).

### C1(c):

### Crystal structure of the α-Polymorph of N-piperidinyl-5-(4-chlorophenyl)-1-(2,4-dichlorophenyl)-4,5-dihydro-1H-pyrazole-3-carboxamide

Crystals suitable for X-ray single crystal determination could be obtained from methanol and ethanol (also dimethylformamide) by slow evaporation at room temperature. Typically the α-polymorph of N-piperidinyl-5-(4-chlorophenyl)-1-(2,4-dichlorophenyl)-4,5-dihydro-1H-pyrazole-3-carboxamide grows in form of twinned needles.

A selected crystal was tested using an X-ray single crystal structure determination.

### Procedure:

The measured crystals were selected using a Zeiss stereomicroscope using polarized light and prepared under inert conditions immersed in perfloropolyether as protecting oil for manipulation. Crystal structure determination was carried out using a Bruker-Nonius diffractometer equipped with a APPEX 2 4K CCD area detector, a FR591 rotating anode with Mo_{kα} radiation, Montel mirrors as monochromator and a Kyroflex low temperature device (T = 100K). Fullsphere data collection omega and phi scans.Analysis was done with software.

The α-Polymorph of N-piperidinyl-5-(4-chlorophenyl)-1-(2,4-dichlorophenyl)-4,5-dihydro-1H-pyrazole-3-carboxamide crystallizes in a triclinic cell.

A summary of the cell constants is given in table 1 below:

| | Unit cell dimensions | |
|---|---|---|
| Triclinic *P* 1 | a = 9.6749(11) Å | α = 78.112(3)°. |
| Volume: 2121.3(4) Å³ | b = 13.1273(17) Å | β = 74.449(3)° |
| Density: 1.415 Mg/m³ | C = 17.725 (2) Å | γ = 88.447(3)° |

### Compound Example C2:

### α-Polymorph of (R)-N-piperidinyl-5-(4-chlorophenyl)-1-(2,4-dichlorophenyl)-4,5-dihydro-1H-pyrazole-3-carboxamide

### C2(a):

A solution of (R)-N-piperidinyl-5-(4-chlorophenyl)-1-(2,4-dichlorophenyl)-4,5-dihydro-1H-pyrazole-3-carboxamide in 300 mL of diisopropyl ether was stirred at 450 rpm and heated to a gentle reflux (68°C) under nitrogen athmosphere. To the resulting mixture, diisopropyl ether was added dropwise (2 hours) to dissolve the solid, and the resulting mixture solution was cooled at 20°C in 90 minutes. A white solid precipitated on cooling. The suspension was left 3 hours at 20°C under a gentle stirring (200 rpm), cooled to 0°C in 2 hours and left overnight at that temperature. The crystals formed were filtered off to give white solid wich correspond to the crystalline phase of the α-Polymorph of (R)-N-piperidinyl-5-(4-chlorophenyl)-1-(2,4-dichlorophenyl)-4,5-dihydro-1H-pyrazole-3-carboxamide.

### C2(b):

### Crystal structure of the α-Polymorph of (R)-N-piperidinyl-5-(4-chlorophenyl)-1-(2,4-dichlorophenyl)-4,5-dihydro-1H-pyrazole-3-carboxamide

The crystals of the α-Polymorph are growing in form of needles which are partially twinned. On a crystal an X-ray single crystal structure determination was done. The α-polymorph crystallizes in a monoclinic cell. A summary of the cell constants is given in table 1:

**Table 1: Cell constants of α-polymorph at 100 K**

| | Unit cell dimensions | |
|---|---|---|
| Monoclinic *P*2₁ | a = 13.6460(13) Å | α = 90°. |
| Volume: 2158.8(4) Å³ | b = 11.6896(12) Å | β = 104.519(2)°. |
| Density: 1.390 Mg/m³ | c = 13.9800(14) Å | γ = 90° |

### Compound Example C3:

### Amorphous Phaseof (R)-N-piperidinyl-5-(4-chlorophenyl)-1-(2,4-dichlorophenyl)-4,5-dihydro-1H-pyrazole-3-carboxamide

### C3(a):

The amorphous phase is obtained by the following way:
A sample of (R)-N-piperidinyl-5-(4-chlorophenyl)-1-(2,4-dichlorophenyl)-4,5-dihydro-1H-pyrazole-3-carboxamide is heated to 80°C (a temperature above the melting point of 75-78 °C see above) until it is completely melted. Subsequently it is cooled to room temperature until it is completely solid again to yield the amorphous phase of (R)-N-piperidinyl-5-(4-chlorophenyl)-1-(2,4-dichlorophenyl)-4,5-dihydro-1H-pyrazole-3-carboxamide.

### C3(b):

### DSC analysis of the amorphous phase of (R)-N-piperidinyl-5-(4-chlorophenyl)-1-(2,4-dichlorophenyl)-4,5-dihydro-1H-pyrazole-3-carboxamide

The DSC analysis of the *amorphous phase* presents a base line step between 60 and 70°C corresponding to the glass transition (Tg). No other peak is observed at higher temperatures, indicating that, at least during the analysis, this phase does not crystallize. Since the Tg is a reversible phenomenon, it can be better measured if first the sample is heated to a temperature above the Tg, is then cooled at the same rate to room temperature, and is again heated above the Tg. The true onset of the Tg for the amorphous phase is at 63-66°C.

### Compound Example C4:

### γ-Polymorph of (R)-N-piperidinyl-5-(4-chlorophenyl)-1-(2,4-dichlorophenyl)-4,5-dihydro-1H-pyrazole-3-carboxamide

### C4(a):

A solution of (R)-N-piperidinyl-5-(4-chlorophenyl)-1-(2,4-dichlorophenyl)-4,5-dihydro-1H-pyrazole-3-carboxamide in 300 mL of diisopropyl ether was stirred at 450 rpm and heated to a gentle reflux (68°C) under nitrogen athmosphere. To the resulting mixture, 185 mL diisopropyl ether was added dropwise (2 hours) to dissolve the solid, and the resulting mixture solution was cooled at 20°C in 90 minutes. A white solid precipitated on cooling. The suspension was left 3 hours at 20°C under a gentle stirring (200 rpm), cooled to 0°C in 2 hours and left overnight at that temperature. The crystals formed were filtered off to give white solid (14.99 g, 83% yield) wich correspond to the crystalline phase of the γ-Polymorph of (R)-N-piperidinyl-5-(4-chlorophenyl)-1-(2,4-dichlorophenyl)-4,5-dihydro-1H-pyrazole-3-carboxamide.

### C4(b):

### Crystal structure of the γ-Polymorph of (R)-N-piperidinyl-5-(4-chlorophenyl)-1-(2,4-dichlorophenyl)-4,5-dihydro-1H-pyrazole-3-carboxamide

The crystals of the γ-Polymorph are growing in form of solid blocks. After selecting a crystal fragment an X-ray single crystal structure determination was done. The measurement of the crystal was done at room temperature. The γ-polymorph crystallizes in a monoclinic cell with a β-angle of 92.15°. The orthorhombic disposition, which would mean a β-angle of exactly 90°, gives a non acceptable statistic. A summary of the cell constants is given in table 1:

**Table 1: Cell constants of γ-polymorph at room temperature**

| | Unit cell dimensions | |
|---|---|---|
| Monoclinic *P*2₁ | a = 9.7447(5) Å | α = 90°. |
| Volume: 2200.80(19) Å³ | b = 18.8735(9) Å | β = 92.1500(10)°. |
| Density: 1.363 Mg/m³ | c = 11.9747(6) Å | γ = 90° |

Additionally, the cell dimensions of the γ-polymorph were determined at 100 K (see table 2).

**Table 2: Cell constants of γ-potymorph at 100 K**

| | Unit cell dimensions | |
|---|---|---|
| Monoclinic *P2*₁ | a = 9.719(4) Å | α = 90°. |
| Volume: 2158(3) Å³ | b = 18.683(7) Å | β = 92.41(2)°. |
| Density: 1.390 Mg/m³ | c = 11.896(4) Å | γ = 90° |

## Claims

1. Solid dispersion and/or solid solution of one or more active principles selected from compounds of formula (I), (Ia) or (Ib) or mixtures thereof 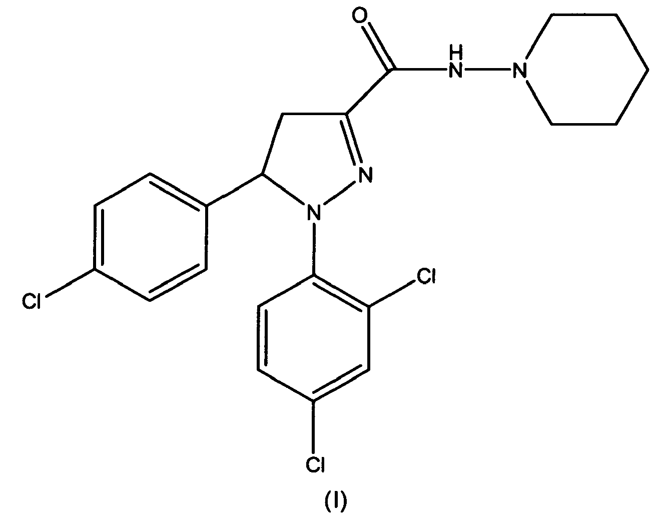 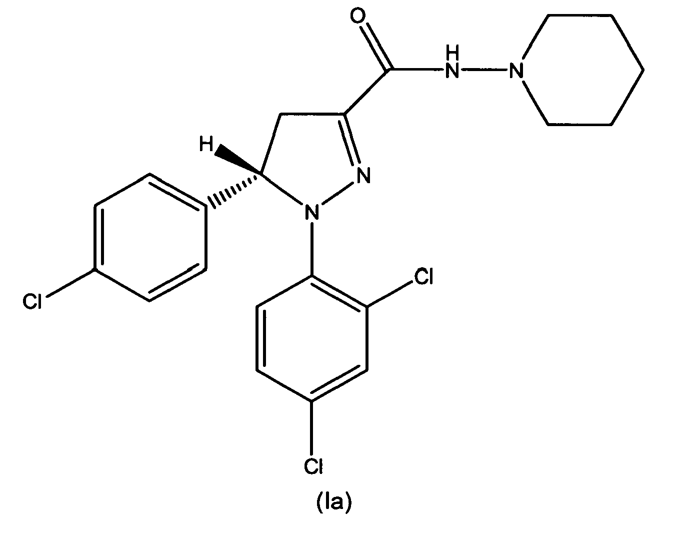 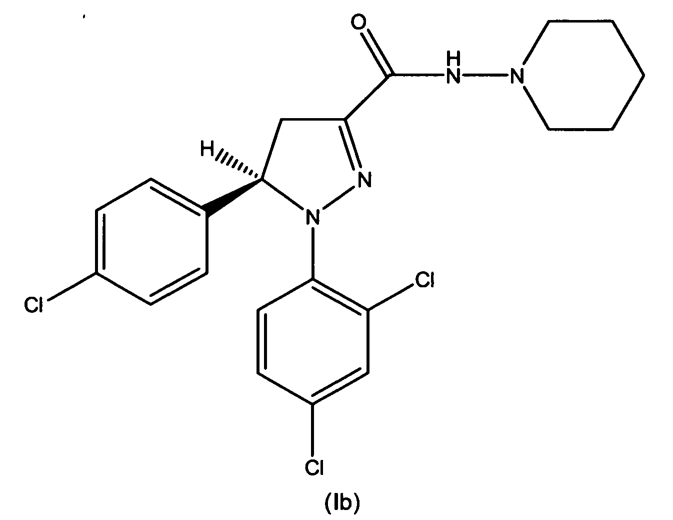 , optionally in form of one or more of the appropriate polymorphs, in amorphous form and/or corresponding salts and/or corresponding solvates thereof,
obtainable by a process in which
a) the active principle/s is/are mixed with an at least equimolar amount of at least one carrier,
b) thereafter or during step a) the mixture is heated until the mixture is melted and
c) subsequently the mixture is cooled below the melting point.

2. Solid dispersion and/or solid solution according to claim 1, **characterized in that** the carrier is either hydrophilic or hygroscopic and has a melting point between +40°C and the melting point of the active principle or mixture of active principles +20 °C.

3. Solid dispersion and/or solid solution according to claim 1, **characterized in that** the carrier is selected from
• sugars, like dextrose, sucrose, galactose, sorbitol, maltose, xylitol, mamitol, lactose;
• acids, like citric acid, succinic acid;
• polymeric materials, like povidone (PVP), polyethylene oxide, polyethylene glycol (PEG), hydroxpropylmethylcellulose, methylcellulose, ethylcellulose hydroxyethylcelllose, cylodextrin, hydroxypropylcellulose, pectin, galactomannan;
• insoluble or enteric polymers, like hydroxypropylmethylcellulose phthalate, polymethacrylates (e.g. Eudragit L-100, Eudragit S-100, Eudragit RL, Eudragit RS);
• surfactants, like polyoxyethylene stearate, renex, poloxamer 188, texafor, AIP, deoxycholic acid, polyoxy-ethylene-sorbitan higher fatty acid esters (e.g. Tween, like Tween 80), spans;
• others, consisting of: camuba wax, pentaerythritol, pentaerythrityltetraacetate, urea, urethane, hydroxyalkylxanthins;
preferably the carriers are selected from EUDRAGIT, MYRJ 52, VITE-TPGS, GELUCIRE 50/13, HPMC-PHTALATE, HPMC, HEC, HPC-SL, PEO and/or POLOXAMER.

4. Solid dispersion and/or solid solution according to any of claims 1 to 3, **characterized in that**
• the melting temperature in step (b) is in the range between +40°C and the melting point of the active principle or mixture of active principles +20 °C; and/or
• the cooling step (c) is done by lowering the temperature by more than 10°C/sec, preferably 20°C/sec, down to temperatures below 25 °C, preferably below 0 °C; and/or is done by contacting the melt of step (b) with an environment having a temperature of 25°C or lower, preferably 0°C or lower.

5. Solid dispersion and/or solid solution according to claim 1, **characterized in that** the molecular ratio between carrier and active principle is between 1:1 and 1:20, preferably is between 1:1 and 1:10, more preferably is between 1:2 and 1:5.

6. A pharmaceutical composition comprising the solid solution and/or solid dispersion according to any of claims 1 to 5 and optionally further pharmaceutical acceptable ingredients.

7. A pharmaceutical composition according to claim 6, **characterized in that**
• the active principle is present in an amount of 10 to 60, preferably 20 to 40 % by weight based on the total weight of the composition; and/or
• the active principle is present in an amount of 1 to 250 mg, preferably 10 to 200 mg, more preferably 15 to 150 mg in the composition.

8. A pharmaceutical composition comprising the solid solution according to any of claims 6 or 7, **characterized in that** it is suitable to enhance the absorption of the active principle through a mucosa.

9. A pharmaceutical composition according to claim 8, **characterized in that** the mucosa is selected from the
• nasal or olfactory mucosa,
• buccal or oral mucosa,
• gastric mucosa,
• intestinal mucosa
• vaginal mucosa, or
• rectal mucosa,
preferably selected from
• gastric mucosa, or
• intestinal mucosa.

10. A pharmaceutical composition according to any of claims 6 to 9, **characterized in that** the pharmaceutical composition is selected from
• a pharmaceutical composition for oral application,
• a pharmaceutical composition for nasal application,
• a pharmaceutical composition for buccal application,
• a pharmaceutical composition for rectal application, or
• a pharmaceutical composition for vaginal application;
or
• a pharmaceutical composition for transdermal application;
• a pharmaceutical composition for systemic application;
preferably selected from
• a pharmaceutical composition for oral application.

11. A pharmaceutical composition according to any of claims 6 to 10, **characterized in that** the pharmaceutical composition is selected from
• a tablet,
• a capsule,
• a sachet,
• a powder,
• a caplet,
• a gel,
• a film,
• a pellet,
• a granule,
• an implant,
• a multiparticulate with granules compressed into a tablet,
• a multiparticulate with granules filled into a capsule,
• a multiparticulate with pelets compressed into a tablet,
• a multiparticulate with pelets filled into a capsule, or
• a suppository,
or
• an injectable solution/dispersion
• a transdermal system like a patch;
preferably selected from
• a tablet,
• a capsule,
• a pellet,
• a granule,
• a multiparticulate with granules compressed into a tablet,
• a multiparticulate with granules filled into a capsule,
• a multiparticulate with pelets compressed into a tablet, or
• a multiparticulate with pelets filled into a capsule.

12. A pharmaceutical composition according to any of claims 6 to 11, **characterized in that**
• the further pharmaceutical acceptable ingredients are present in a total amount of 5 to 95 %, preferably 50 to 90 % by weight based on the total weight of the composition;
and/or
• the further pharmaceutical acceptable ingredients are present in a total amount of 0 to 300 mg, preferably 0 to 250 mg, more preferably 20 to 250 mg.

13. A pharmaceutical composition according to any of claims 6 to 12, **characterized in that** the further pharmaceutical acceptable ingredient/s is/are selected from a diluent, a binder and/or a lubricant, and/or optionally a flowing agent, an antiadhesive, a preservative, and/or, optionally, a taste masking agent, a coloring agent and/or a flavoring agent and/or a permeation enhancer.

14. A pharmaceutical composition according to any of claims 6 to 13, **characterized in that** the further composition is prepared using hot-melt extrusion.

15. A pharmaceutical composition comprising as active principle at least one of
• (rac)-5-(4-Chloro-phenyl)-1-(2,4-dichloro-phenyl)-4,5-dihydro-1H-pyrazole-3-carboxylic acid piperidin-1-ylamide;
• (R)- 5-(4-Chloro-phenyl)-1-(2,4-dichloro-phenyl)-4,5-dihydro-1H-pyrazole-3-carboxylic acid piperidin-1-ylamide, or
• (S)- 5-(4-Chloro-phenyl)-1-(2,4-dichloro-phenyl)-4,5-dihydro-1H-pyrazole-3-carboxylic acid piperidin-1-ylamide,
• or mixtures thereof;
optionally in form of one or more of the appropriate polymorphs, in amorphous form and/or corresponding salts and/or corresponding solvates thereof,
in a solid dispersion and/or solid solution in a carrier, the carrier having a melting point between +40°C and +20 °C above the melting point of the active principle and being hydrophilic or hygroscopic;
and optionally further pharmaceutical acceptable ingredients

16. A pharmaceutical composition for oral administration comprising the solid solution and/or solid dispersion according to any of claims 1 to 5 and optionally further pharmaceutical acceptable ingredients.

17. A solid dispersion and/or solid solution according to any of claims 1 to 5 or a pharmaceutical composition according to any of claims 6 to 15 and 16, **characterized in that** the active principle is selected from
a) (rac)-5-(4-Chloro-phenyl)-1-(2,4-dichloro-phenyl)-4,5-dihydro-1H-pyrazole-3-carboxylic acid piperidin-1-ylamide;
preferably in
• form of the α-polymorph;
• form of the β-polymorph;
• amorphous form;
• form of a solvate, preferably a hydrate, more preferably a monohydrate;
• the free base; or
• a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1 ,5-disulfonic acid, nitric acid, phosophoric acid, p-toluenesulfonic acid, sulfuric acid and/or thiocyanic acid; more preferably the salt being a hydrochloride;
b) (R)-5-(4-Chloro-phenyl)-1-(2,4-dichloro-phenyl)-4,5-dihydro-1H-pyrazole-3-carboxylic acid piperidin-1-ylamide, preferably
preferably in
• form of the α-polymorph;
• form of the γ-polymorph;
• amorphous form;
• form of a solvate, preferably a hydrate,
• the free base;
• a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, p-toluenesulfonic acid, sulfuric acid and/or thiocyanic acid; more preferably the salt being a hydrochloride;
c) (S)- 5-(4-Chloro-phenyl)-1-(2,4-dichloro-phenyl)-4,5-dihydro-1H-pyrazole-3-carboxylic acid piperidin-1-ylamide,
preferably in
• form of a polymorph;
• amorphous form;
• form of a solvate, preferably a hydrate,
• the free base;
• a salt with an acid with a pkₐ ≤ 3.0,especially the acid being selected from 2,5-dihydroxybenzenesulfonic acid, 2-naphthalenesulfonic acid, aspartic acid, benzenesulfonic acid, amphor-10-sulfonic acid, cyclohexylsulfamic acid, dodecylsulfuric acid, ethane-1,2-disulfonic acid, ethanesulfonic acid, fumaric acid, glutamic acid, hydrobromic acid, hydrochloric acid, methanesulfonic acid, naphthalene-1,5-disulfonic acid, nitric acid, phosophoric acid, *p*-toluenesulfonic acid, sulfuric acid and/or thiocyanic acid; more preferably the salt being a hydrochloride;
or
d) a nonracemic mixtures of (b) and (c).
